# FASCICULE DE BREVET EUROPEEN

(11) **EP 0 502 110 B1**
(45) Date de publication et mention de la délivrance du brevet: **01.02.1995**
(21) Numéro de dépôt: 91900836.7
(22) Date de dépôt: 23.11.1990
(51) Int. Cl.: C07D 257/04, C07D 271/10, C07D 401/12, C07C 243/38, A61K 31/41

(54) **DERIVES D'(HETERO) ARYLMETHYLOXY-4 PHENYL DIAZOLE, LEUR PROCEDE DE PREPARATION ET LEUR APPLICATION EN THERAPEUTIQUE**
4-(HETERO)ARYLMETHYLOXY-PHENYL-DIAZOLDERIVATE, VERFAHREN ZU DEREN HERSTELLUNG UND IHRE THERAPEUTISCHE VERWENDUNG
(HETERO) 4-ARYLMETHOXY PHENYL DIAZOLE DERIVATIVES, METHOD FOR PREPARING SAME, AND THEIR THERAPEUTICAL APPLICATIONS

(30) Priorité: 24.11.1989 FR 8915499
(43) Date de publication de la demande: 09.09.1992
(73) Titulaire: SYNTHELABO, 92350 Le Plessis Robinson (FR)
(72) Inventeur: KOENIG, Jean-Jacques, F-78600 Maisons-Laffitte (FR); LEBRETON, Luc, F-21000 Dijon (FR); MASSON, Maryse, F-75009 Paris (FR)
(74) Mandataire: Kedinger, Jean-Paul
(86) Numéro de dépôt international: FR9000847
(87) Numéro de publication internationale: WO9108201

(56) Documents cités:
- FR-A- 2 001 971
- FR-A- 2 631 827
- FR-M- 8 005
- US-A- 3 865 570
- Chimie Thérapeutique, vol. 4, no. 3, mai-juin 1969, C. Breysse et al.: "Psychotropes potentiels. V. Synthèse et étude pharmacologique de nouvelles hydrazines inhibitrices des monoamine-oxydases et contenant le radical: alcoyloxy-4 dihalogéno-3,5 benzoyle", pages 157-166
- Chemical Abstracts, vol. 113, no. 12, 17 september 1990, (Columbus, Ohio, US) page 674, résumé 107010g, & JP, A, 0201482 (TOSHIBA CORP.; FUJISAWA PHARMACEUTICAL CO. LTD), 5 janvier 1990

## Description

La présente invention a pour objet de nouveaux dérivés d'(hétéro) arylméthyloxy-4 phényl diazole, leur procédé de préparation et leur application en thérapeutique.

Par FR-A-2 631 827, on connaît certains dérivés d'(hétéro)aryl-5 tétrazole ; par Chem. Abst., vol.113, No 12, 17/9/90, page 674, résumé 107010g et JP-A-9001482, on connaît certaines aryl-1,2,4-triazines et aryl-1,3,4-thiadiazoles ainsi la [benzyloxy-4 benzoyl]-1 hydrazine ; et par Chimie Thérapeutique, vol.4, No 3, mai-juin 1969, C. Breysse et al., on connaît des dérivés hydraziniques d'alcoyloxy-benzoyle.

Les dérivés selon l'invention répondent plus précisément à la formule : 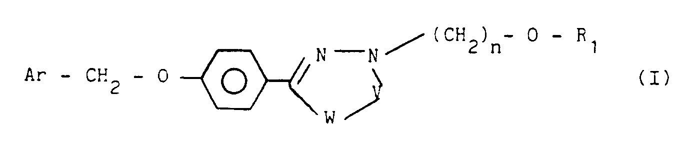
dans laquelle R₁ = alkyle en C₁-C₄ et Ar est un groupe aryle ou hétéroaryle choisi parmi les suivants :
(i) 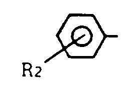 où R₂ représente un atome d'hydrogène, un ou deux atomes d'halogène, un groupe CN, NO₂ ou CF₃, un, deux ou trois groupes alkyle en C₁-C₄ ou alkoxy en C₁-C₄, ou un groupe amino substitué par deux groupes alkyle en C₁-C₄, auquel cas l'enchaînement -W-V- représente -N = N- ou 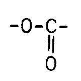 et n = 2-6 ;
(ii) pyridyle auquel cas l'enchaînement -W-V- représente -N=N- et n = 1-6.

Dans ce qui précède et qui suit, l'expression "alkyle en C₁-C₄" désigne des groupes hydrocarbonés, linéaires ou ramifiés, comprenant 1 à 4 atomes de carbone, à savoir méthyle, éthyle, n-propyle, i-propyle, n-butyle, i-butyle, sec.-butyle et tert-butyle ; l'expression "alkoxy en C₁-C₄" possède la formule O-alkyle en C₁-C₄ ; le terme halogène désire fluor, chlore, brome et iode ; et le terme pyridyle désigne pyridyle-4, pyridyle-3 ou pyridyle-2.

La présente invention vise également les sels d'addition d'acide minéral (tel l'acide chlorhydrique ou sulfurique par exemple) ou organique (tel l'acide acétique, oxalique, maléique ou tartrique par exemple) des dérivés (I) salifiables.

Elle s'étend en outre au procédé de préparation des dérivés (I), ce procédé comprenant :
a) la condensation des composés de formule :

   X - (CH₂)ₙ - OR₁ (II)

   où n = 2-6, R₁ = alkyle en C₁-C₄ et X représente un groupe bon partant tel qu'un atome d'halogène (chlore, brome ou iode) ou un groupe mésyloxy ou tosyloxy, respectivement sur les composés de formule : 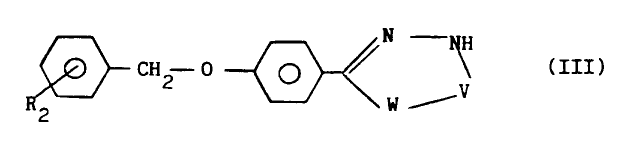 où -W-V- représente -N=N- ou 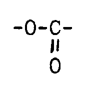 et R₂ a la même signification que dans la formule (I), en présence d'une base comme un hydrure métallique, notamment l'hydrure de sodium, dans un solvant anhydre aprotique comme le diméthylformamide ;
b) la condensation des composés de formule :

   Ar - CH₂ - X (IV)

   où Ar a la même signification que dans la formule (I) et X a la même signification que dans la formule (II), respectivement sur les composés de formule : 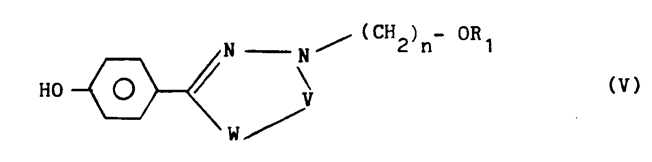 où R₁ et n ont la même signification que dans la formule (I) et -W-V- représente soit -N=N- ou 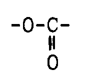 quand Ar dans la formule (IV) est un noyau phényle substitué par R₂, ce dernier ayant la même signification que dans la formule (I), soit -N=N- quand Ar dans la formule (IV) représente pyridyle, en présence d'une base comme K₂CO₃ ou d'un hydrure métallique comme l'hydrure de sodium, dans un solvant anhydre comme la DMF ou l'acétonitrile ;
c) la condensation des composés de formule : 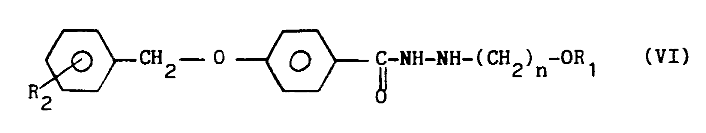 où n, R₁ et R₂ ont la même signification que dans la formule (I), avec du phosgène dans un solvant aprotique comme le dioxanne, le chloroforme ou le toluène, ce qui permet la cyclisation de la chaîne 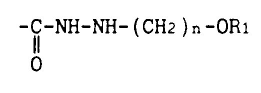 des composés

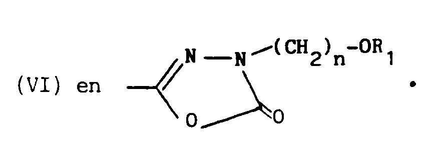

Les composés de formule (V) peuvent être obtenus par débenzylation en présence d'hydrogène et d'un catalyseur comme le palladium sur carbone, respectivement des composés de formule (I) de structure particulière : 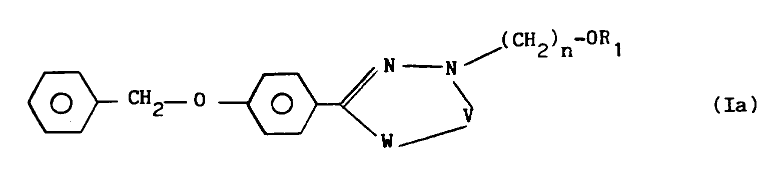
où -W-V-, R₁ et n ont la même signification que dans la formule (I), de préférence dans un solvant alcoolique notamment l'éthanol ou le méthanol.

Les composés de formule (III) et de structure particulière : 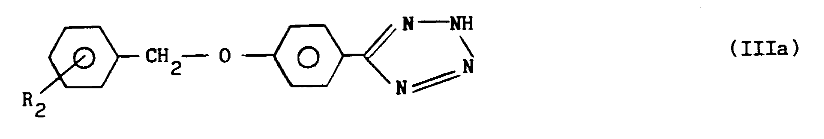
où R₂ a la même signification que dans la formule (I) peuvent être obtenus par action de l'azoture de sodium sur les composés de formule : 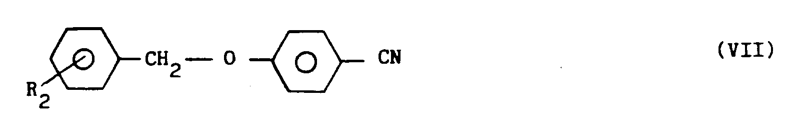
où R₂ a la même signification que dans la formule (I), de préférence selon la méthode de R.M. HERBST décrite dans J. Org. Chem. 22, 1142, 1957.

Les composés de formule (VII) sont obtenus en traitant le composé de formule : 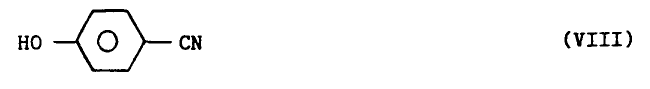
par un hydrure métallique comme l'hydrure de sodium dans un solvant aprotique tel le DMF puis en faisant réagir le composé ainsi obtenu avec les composés de formule (IV) pour lesquels

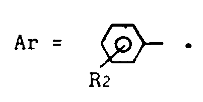

Les composés de formule (III) de structure particulière 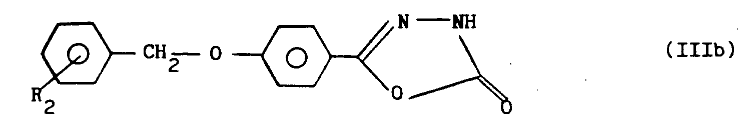
où R₂ a la même signification que dans la formule (I) peuvent être obtenus selon la méthode de FREUND et GOLDSMITH décrite dans Ber. 21, 1240, 1882, par mise en oeuvre d'hydrazides de formule : 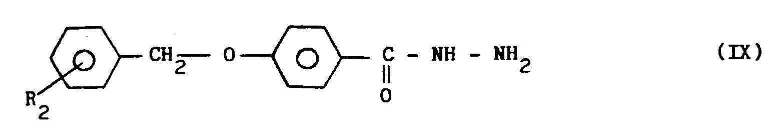
où R₂ a la même signification que dans la formule (I).

Les composés de formule (IX) peuvent être obtenus par action de l'hydrazine respectivement sur l'ester éthylique ou méthylique des acides de formule : 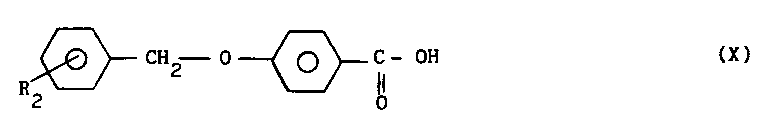
où R₂ a la même signification que dans la formule (I) en milieu alcoolique de préférence l'éthanol.

Les composés de formule (VI) sont obtenus par action de l'ester méthylique ou éthylique des acides de formule (X) en milieu alcoolique comme l'éthanol ou le méthanol, respectivement sur les composés de formule :

NH₂ - NH - (CH₂)ₙ - OR₁ (XI)

où n et R₁ ont la même signification que dans la formule (I), eux-mêmes obtenus préférentiellement par la méthode décrite dans CA 63 : 9962.

Quant aux sels d'addition d'acide des dérivés salifiables de formule (I), ils peuvent être obtenus de manière tout à fait classique par action d'un acide minéral ou organique sur les dérivés salifiables de formule (I), ces acides et dérivés étant de préférence utilisés sous la forme de solutions miscibles.

Les préparations suivantes sont données à titre d'exemples pour illustrer l'invention.

### Exemple 1 : (benzyloxy-4 phényl)-5 méthoxyéthyl-2 2H tétrazole [(I) ; n = 2, R₁ = CH₃, -W-V-:-N=N-, Ar = phényle]

Numéro de code : MD 230300

### 1ère étape : benzyloxy-4 benzonitrile (VII)

A une solution de 5.10⁻² mole d'hydroxy-4 benzonitrile dans 100 ml de DMF, on ajoute petit à petit, 5.10⁻² mole de NaH de façon à obtenir une température de 25° C. Puis la solution est chauffée à 50° C jusqu'à cessation du dégagement d'hydrogène. Après refroidissement à 0° C, on ajoute 5.10⁻² de chlorure de benzyle. Le mélange réactionnel est chauffé à 40° C pendant 1 heure, puis versé dans 300 ml d'eau glacée. Le solide résultant est séparé par filtration.

### 2ème étape : (benzyloxy-4 phényl)-5 tétrazole [(IIIa);R₂=H]

A une solution de 5.10⁻² mole de benzyloxy-4 benzonitrile (VIII) dans 20 ml de butanol, on ajoute 6,6.10⁻² mole d'azoture de sodium et 6,6.10⁻² mole d'acide acétique. Puis le milieu réactionnel est chauffé au reflux pendant 4 jours. On ajoute 1 g d'azoture de sodium, 2 g d'acide acétique et 10 ml de butanol et le milieu réactionnel est à nouveau chauffé au reflux 2 jours. Après concentration par évaporation du solvant, le résidu est repris dans 20 ml de NaOH aqueuse à 10 %. Après filtration, la phase aqueuse est extraite à l'éther. Par acidification de la solution alcaline par HCl 2N, le composé attendu est isolé avec un rendement de 85 % (F = 228° C).

### 3ème étape : (benzyloxy-4 phényl)-5 méthoxyéthyl-2 2H tétrazole (I)

A une solution de 2.10⁻² mole d'hydrure de sodium dans 50 ml de DMF, on ajoute 2.10⁻² mole du composé obtenu à la 2ème étape et on chauffe à 60-80° C le milieu réactionnel jusqu'à cessation du dégagement gazeux. Après refroidissement, on ajoute, petit à petit, 2.10⁻² mole de chloro-2 méthoxy-1 éthane. Le milieu réactionnel est chauffé à 80° C pendant 12 heures, puis concentré au 2/3 et versé sur de l'eau glacée. Après extraction à l'éther, le produit attendu est obtenu avec un rendement de 48 %.
. F = 96° C
. IR (KBr, ν cm⁻¹) : 1610, 1450
. ¹H RMN ( δ ppm, DMSOd₆) : 3,2 (3H) ; 3,9 (2H) ; 5,3 (2H) ; 7,2 (2H) ; 7,4 (5H) ; 8 (2H).

On peut de la même manière obtenir les autres dérivés de formule (I) pour lesquels 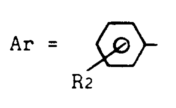
et notamment les suivants :
- [(chloro-4 benzyloxy)-4 phényl]-5 méthoxyéthyl-2 2H-tétrazole [(I) ; -W-V-:-N=N-, R₁ = CH₃, n = 2, Ar = chloro-4 phényle] Numéro de code : MD 230305
   . Rendement = 44 %
   . F = 90° C
   . IR (KBr, ν cm⁻¹) ; 1620, 1420, 1410, 1210
   . ¹H RMN ( δ ppm, DMSOd₆) : 3,2 (3H) ; 3,9 (2H) ; 4,9 (2H) ; 5,2 (2H) ; 7,2 (2H) ; 7,5 (4H) ; 8 (2H) ;
- [(chloro-3 benzyloxy)-4 phényl]-5 méthoxyéthyl-2 2H tétrazole
   [(I) ; -W-V-:-N=N-, R₁ = CH₃, n = 2, Ar = chloro-3 phényle]
   Numéro de code : MD 230324
   . F = 92° C
   . ¹H RMN ( δ ppm, DMSOd₆) : 3,4 (3H) ; 4 (2H) ; 4,8 (2H) ; 5,1 (2H) ; 7 (2H) ; 7,3 (4H) ; 8 (2H).

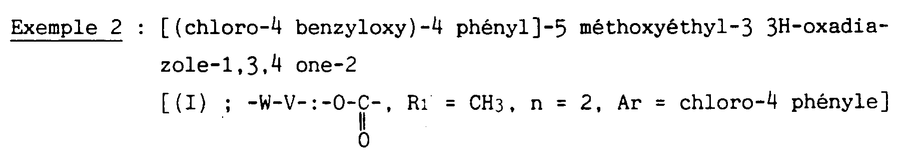

Numéro de code : MD 230306

### 1ère étape : [(chloro-4 benzyloxy)-4 benzoyl]-1 hydrazine (IX)

A une solution de 0,275 mole d'ester éthylique de l'acide (chloro-4 benzyloxy)-4 benzoïque (X) dans 300 ml d'éthanol, on ajoute 2,75 mole d'hydrate d'hydrazine. Le milieu réactionnel est chauffé 48 heures à reflux. Après refroidissement le produit attendu obtenu est séparé par filtration et recristallisé dans l'éthanol.
. Rendement = 63 %
. F = 168° C

### 2ème étape : [(chloro-4 benzyloxy)-4 phényl]-5 3H oxadiazole-1,3,4 one-2 (IIIb)

A une solution de 0,0723 mole du composé obtenu à l'étape précédente dans 200 ml de toluène, on ajoute une solution de 0,0723 mole de phosgène dans 5 ml de toluène. Le mélange est agité pendant 18 heures à température ambiante. Après filtration, le produit obtenu est recristallisé dans le butanol.
. Rendement = 78 %
. F = 236° C

### 3ème étape : [(chloro-4 benzyloxy)-4 phényl]-5 méthoxyéthyl-3 3H oxadiazole-1,3,4 one-2 (I)

A une solution de 0,016 mole du composé obtenu à l'étape précédente dans 50 ml de DMF, on ajoute 0,016 mole de NaH et chauffe à 60-80° C jusqu'à cessation du dégagement d'hydrogène. Après refroidissement, on ajoute, goutte à goutte, 0,016 mole de chloro-2 méthoxy-1 éthane, puis le milieu réactionnel est chauffé à 80° C pendant 12 heures, puis concentré au 2/3 et versé sur de l'eau glacée. Par extraction à l'éther, on obtient le produit attendu avec un rendement de 36 %.
F = 140° C
. IR (KBr, ν cm⁻¹) : 1780, 1610, 1245, 1115, 1000.
. ¹H RMN (DMSOd₆) δ ppm : 3,2 (3H) ; 3,8 (4H) ; 5,2 (2H) ; 7,2 (2H) ; 7,5 (4H) ; 7,7 (2H).

### Exemple 3 : chlorhydrate de méthoxyéthyl-2 [(pyridyl-4 méthyloxy)-4 phényl]-5 2H tétrazole [(I) ; -W-V-:-N=N-, n = 2, R₁ = CH₃, Ar = pyridyl-4]

Numéro de code : MD 230307

### 1ère étape : (hydroxy-4 phényl)-5 méthoxyéthyl-2 2H tétrazole [(V) : -W-V-:-N=N-, n=2, R₁=CH₃]

A une solution de 0,6.10⁻³ mole du composé MD 230300 dans 20 ml d'un mélange de méthanol et de chlorure de méthylène (50-50), on ajoute 0,04 g de palladium sur carbone à 10 % humidifié à 50 % et fait arriver un courant d'hydrogène sous pression normale pendant 3 heures. Après filtration et concentration, le produit obtenu est recristallisé dans un mélange d'éther éthylique et d'éther de pétrole.
. Rendement = 75 %
. F = 110° C

### 2ème étape : chlorhydrate de méthoxyéthyl-2 [(pyridyl-4 méthyloxy)-4 phényl]-5 2H tétrazole (I)

A une solution de 0,0227 mole du composé obtenu à l'étape précédente dans 100 ml d'acétonitrile, on ajoute 0,0567 mole de K₂CO₃ et 0,0227 mole de chlorométhyl-4 pyridine. Le milieu réactionnel est chauffé au reflux pendant 48 heures, puis concentré et repris dans une solution de NaOH 0,1 N. Après extraction au chlorure de méthylène, la phase organique est séchée sur sulfate de sodium et concentrée. La base obtenue est dissoute dans l'éthanol, puis on ajoute de l'éthanol chlorhydrique. Le chlorhydrate attendu précipite et on le recristallise dans l'éthanol.
. Rendement = 15 %
. F = 190° C
. IR (KBr, ν cm⁻¹) : 2400, 2100, 2000, 1640, 1615, 1470, 1260, 1250, 1120
. ¹H RMN ( δ ppm, DMSOd₆) : 3,2 (3H) ; 3,9 (2H) ; 4,9 (2H) ; 5,6 (2H) ; 7,2 (2H) ; 8 (4H) ; 8,9 (2H) ; 7,7 (1H éch.)

On obtient de la même manière les autres dérivés (I) et notamment le chlorhydrate de (méthoxyéthyl)-2 [(pyridyl-3 méthyloxy)-4 phényl]-5 2H tétrazole [(I) ; -W-V-:-N=N-, n = 2, R₁ = CH₃, Ar = pyridyle-3] - Numéro de code de code : MD 230308.
. F = 160° C
. IR (KBr, ν cm⁻¹) : 2550, 2100, 1615, 1550, 1420, 1405, 1260
. ¹H RMN ( δ ppm, DMSOd₆) : 3,3 (3H) ; 4 (2H) ; 4,95 (2H) ; 5,5 (2H) ; 7,3 (2H) ; 8 (3H) ; 8,5-8,9 (3H) ; 9,1 (1H éch.)

Les dérivés de formule (I) et leurs sels d'addition d'acide pharmaceutiquement acceptables ont été étudiés chez l'animal de laboratoire et ont montré une activité pharmacologique, notamment une activité sélective inhibitrice de la monoamine oxydase de type B (MAO-B).

L'activité inhibitrice de la monoamine oxydase de ces composés a été mise en évidence par des mesures in vitro de la MAO.

Le dosage de l'activité de la MAO a été effectué en utilisant comme source d'enzyme une suspension mitochondriale du cerveau de rat. Le dosage standard consiste à préincuber l'enzyme pendant 20 minutes en absence, puis en présence des inhibiteurs. Les activités sont déterminées en utilisant la sérotonine (5 HT) et la phénéthylamine (PEA) comme substrats de la MAO-A et de la MAO-B, les temps de réaction étant de 40 minutes avec la 5 HT et de 10 minutes avec la PEA. Le mode opératoire mis en oeuvre est celui du protocole de P.C. Baker : Dev. Biol. 14, 267, 1966.

Les activités d'un certain nombre de composés selon l'invention vis-à-vis de la MAO-B et de la MAO-A sont données par leurs constantes d'inhibition Ki (MAO-B) et Ki(MAO-A) et sont rassemblées dans le tableau ci-après.

**TABLEAU**

| Composé testé Numéro de code | Ki(MAO-B) | Ki(MAO-A) |
|---|---|---|
| MD 230300 | 1,56.10⁻⁸ M/L | inactif |
| MD 230305 | 1,84.10⁻⁷ M/L | " |
| MD 230306 | 5.10⁻⁹ M/L | " |
| MD 230324 | 3,5.10⁻⁸ M/L | " |

En ce qui concerne la toxicité des dérivés de formule (I) et de leurs sels d'addition d'acide pharmaceutiquement acceptables, on notera, qu'après administration à la souris par voie orale, il n'a pas été observé de toxicité pendant 24 heures jusqu'à une dose de 1500 mg/kg.

Les dérivés de formule (I) et leurs sels d'addition d'acide pharmaceutiquement acceptables peuvent donc être utilisés pour la préparation de médicaments inhibiteurs de la monoamine oxydase de type B, ces médicaments trouvant leur emploi en thérapeutique notamment pour le traitement des troubles neurologiques liés au vieillissement pathologique, des troubles mnésiques, de l'humeur, de la schizophrénie, de la psychiasténie ou ralentissement psychique lié au vieillissement, de certaines formes de dépression et de la maladie de Parkinson.

Ces médicaments peuvent être administrés à l'homme ou à tout animal à sang chaud, sous diverses formes pharmaceutiques bien connues dans la technique considérée et notamment sous la forme de compositions formulées en vue de l'administration par voie orale, parentérale ou rectale.

Pour l'administration par voie orale, lesdites compositions peuvent prendre la forme de comprimés, dragées ou gélules, préparés par les techniques habituelles utilisant des supports et excipients connus tels que des agents liants, des charges, des lubrifiants et des agents de désintégration ; elles peuvent également prendre la forme de solutions, de sirop ou de suspensions.

Par l'administration par voie parentérale, les compositions selon l'invention peuvent prendre la forme de solutions, suspensions ou émulsions injectable comprenant un véhicule liquide, huileux ou aqueux, parentéralement acceptable.

Pour l'administration par voie rectale, les compositions peuvent prendre la forme de suppositoires comprenant les bases habituelles pour suppositoires.

La dose à laquelle les principes actifs, c'est-à-dire les dérivés (I) et leurs sels d'addition d'acide pharmaceutiquement acceptables, peuvent être administrés dépendra notamment de la voie d'administration, du poids corporel du patient et de la puissance thérapeutique des composés mis en oeuvre. Généralement par voie orale, les doses pourront atteindre 50 mg/kg de principe actif par jour (en une ou plusieurs prises) ; par voie parentérale les doses pourront atteindre 5 mg/kg de principe actif par jour (en une seule ou plusieurs prises) ; et par voie rectale les doses pourront atteindre 10 mg/kg de principe actif par jour (en un ou deux suppositoires).

## Revendications

1. Dérivés répondant à la formule : 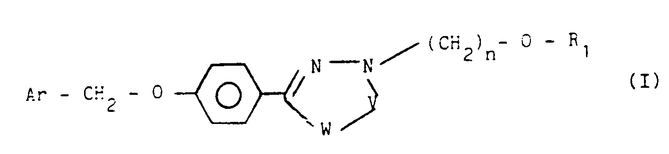 dans laquelle R₁ = alkyle en C₁-C₄ et Ar est un groupe aryle ou hétéroaryle choisi parmi les suivants :
(i) 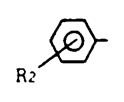 où R₂ représente un atome d'hydrogène, un ou deux atomes d'halogène, un groupe CN, NO₂ ou CF₃, un, deux ou trois groupes alkyle en C₁-C₄ ou alkoxy en C₁-C₄, ou un groupe amino substitué par deux groupes alkyle en C₁-C₄, auquel cas l'enchaînement -W-V- représente -N = N- ou 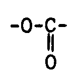 et n = 2-6 ;
(ii) pyridyle auquel cas l'enchaînement -W-V- représente -N=N- et n = 1-6,
ainsi que les sels d'addition d'acide des dérivés (I) salifiables.

2. Dérivé selon la revendication 1, caractérisé en ce qu'il est choisi parmi les suivants :
- (benzyloxy-4 phényl)-5 méthoxyéthyl-2 2H tétrazole,
- [(chloro-4 benzyloxy)-4 phényl]-5 méthoxyéthyl-2 2H tétrazole,
- [(chloro-3 benzyloxy)-4 phényl]-5 méthoxyéthyl-2 2H tétrazole.

3. [(Chloro-4 benzyloxy)-4 phényl]-5 méthoxyéthyl-3 3H oxadiazole-1,3,4 one-2.

4. Dérivé et sel selon la revendication 1, caractérisés en ce qu'ils sont choisis parmi les suivants :
- méthoxyéthyl-2 [(pyridyl-4 méthyloxy)-4 phényl]-5 2H tétrazole et son chlorhydrate,
- méthoxyéthyl-2 [(pyridyl-3 méthyloxy)-4 phényl]-5 2H tétrazole et son chlorhydrate.

5. Composition pharmaceutique, caractérisée en ce qu'elle comprend un dérivé de formule (I) selon l'une quelconque des revendications 1 à 4 ou un sel d'addition d'acide pharmaceutiquement acceptable d'un dérivé salifiable de formule (I) et un support pharmaceutiquement acceptable.

6. Utilisation des dérivés et sels selon l'une quelconque des revendications 1 à 4, pour la fabrication d'un médicament inhibiteur de la monoamine oxydase de type B.

7. Procédé de préparation des dérivés de formule (I) et sels selon l'une quelconque des revendications 1 à 4, caractérisé en ce qu'il comprend :
a) la condensation des composés de formule :
X - (CH₂)ₙ - OR₁ (II)
où n = 2-6, R₁ = alkyle en C₁-C₄, et X représente un groupe bon partant, respectivement sur les composés de formule : 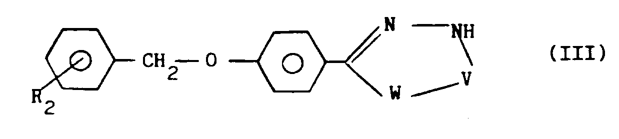 où -W-V- représente -N=N- ou 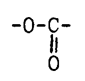 et R₂ a la même signication que dans la formule (I), en présence d'une base,
b) la condensation des composés de formule :
Ar - CH₂ - X (IV)
où Ar a la même signification que dans la formule (I) et X a la même signification que dans la formule (II), respectivement sur les composés de formule : 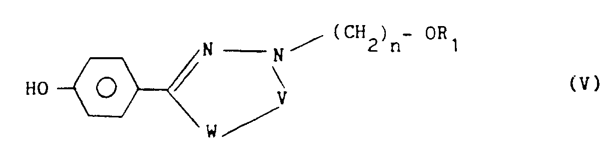 où R₁ et n ont la même signification que dans la formule (I) et -W-V- représente soit -N=N- ou 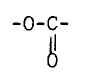 quand Ar dans la formule (IV) est un noyau phényle substitué par R₂, ce dernier ayant la même signification que dans la formule (I), soit -N=N- quand Ar dans la formule (IV) représente pyridyle, en présence d'une base, ou
c) la condensation des composés de formule : 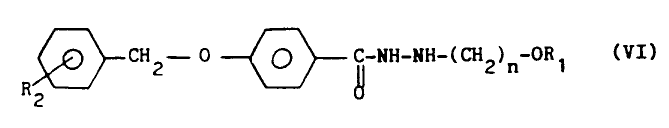 où n, R₁ et R₂ ont la même signification que dans la formule (I), avec du phosgène dans un solvant aprotique.

8. Procédé selon la revendication 7, caractérisé en ce que X dans la formule (II) représente un atome d'halogène ou un groupe mésyloxy ou tosyloxy et en ce que la base mise en oeuvre dans la condensation a) est un hydrure métallique.

9. Procédé selon la revendication 7, caractérisé en ce que la base mise en oeuvre dans la condensation b) est K₂CO₃ ou un hydrure métallique.

10. Composés de formule : 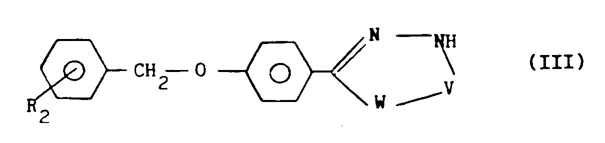 dans laquelle -W-V- représente -N=N- ou 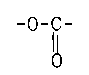 et R₂ représente un atome d'hydrogène, un ou deux atomes d'halogène, un groupe CN, NO₂ ou CF₃, un, deux ou trois groupes alkyle en C₁-C₄ ou alkoxy en C₁-C₄, ou un groupe amino substitué par deux groupes alkyle en C₁-C₄.

11. Composés de formule : 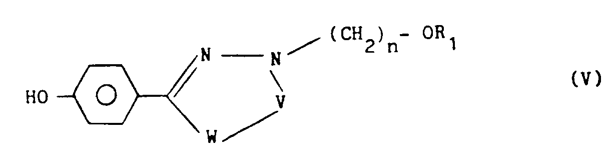 où R₁ = alkyle en C₁-C₄ et n = 1-6.

12. Composés de formule : 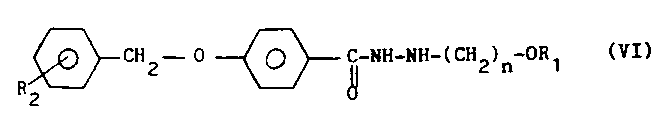 dans laquelle n = 2-6, R₁ = alkyle en C₁-C₄ et R₂ représente un atome d'hydrogène, un ou deux atomes d'halogène, un groupe CN, NO₂ ou CF₃, un, deux ou trois groupes alkyle en C₁-C₄ ou alkoxy en C₁-C₄, ou un groupe amino substitué par deux groupes alkyle en C₁-C₄.

13. Composés de formule : 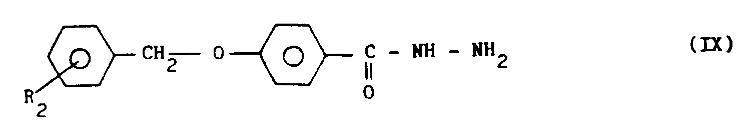 dans laquelle R₂ représente un atome d'hydrogène, un ou deux atomes d'halogène, un groupe CN, NO₂ ou CF₃, un, deux ou trois groupes alkyle en C₁-C₄ ou alkoxy en C₁-C₄, ou un groupe amino substitué par deux groupes alkyle en C₁-C₄ ; à l'exclusion de la [benzyloxy-4 benzoyl]-1 hydrazine.

## Claims

1. Derivatives having the formula: 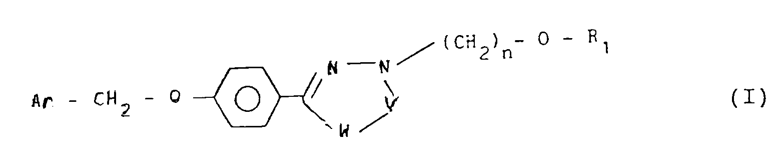 in which R₁ denotes C₁-C₄ alkyl and Ar is an aryl or heteroaryl group chosen from among the following:
(i) 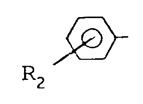 where R₂ represents a hydrogen atom, one or two halogen atoms, a CN, NO₂ or CF₃ group, one, two or three C₁-C₄ alkyl or C₁-C₄ alkoxy groups or an amino group substituted by two C₁-C₄ alkyl groups, in which case the -W-V- chain represents -N = N- or 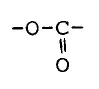 and n = 2-6;
(ii) pyridyl, in which case the -W-V- chain represents -N=N-and n = 1-6,
and acid addition salts of those derivatives (I) which are salt-forming.

2. A derivative according to claim 1, characterised in that it is chosen from among the following:
- 5-(4-benzyloxy phenyl) 2-methoxyethyl 2H tetrazole,
- 5-[4(4-chloro benzyloxy) phenyl] 2-methoxyethyl 2H tetrazole,
- 5-[4-(3-chloro benzyloxy)phenyl] 2-methoxyethyl 2H tetrazole.

3. 5-[4-(4-chloro benzyloxy) phenyl] 3-methoxyethyl 3H 1,3,4-oxadiazol-2-one.

4. A derivative and salt according to claim 1, characterised in that they are chosen from among the following:
- 2-methoxyethyl 5-[4-(4-pyridyl methyloxy)phenyl] 2H tetrazole and the hydrochloride thereof,
- 2-methoxyethyl 5-[4-(3-pyridyl methyloxy)phenyl] 2H tetrazole and the hydrochloride thereof.

5. A pharmaceutical composition characterised in that it comprises a formula (I) derivative according to any of claims 1 to 4 or a pharmaceutically acceptable acid addition salt of a salt-forming derivative of formula (I) and a pharmaceutically acceptable carrier.

6. Use of the derivatives and salts according to any of claims 1 to 4 for manufacturing a drug for inhibiting type B monoamine oxidase.

7. A process of preparing formula (I) derivatives and salts according to any of claims 1 to 4, characterised in that it comprises:
(a) condensation of compounds having the formula:
X - (CH₂)ₙ - OR₁ (II)
where n = 2-6, R₁ = C₁-C₄ alkyl and X represents a good leaving group, on to the respective compounds having the formula 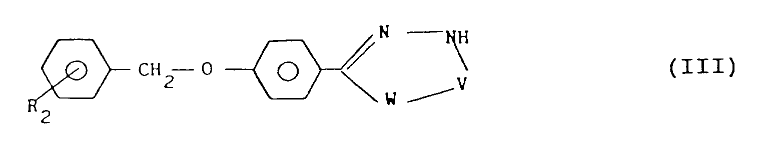 where -W-V- represents -N=N- or 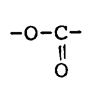 and R₂ have the same meaning as in formula (I), in the presence of a base,
(b) condensation of compounds having the formula:
Ar - CH₂ - X (IV)
where Ar has the same meaning as in formula (I) and X has the same meaning as in formula (II), on to the respective compounds having the formula: 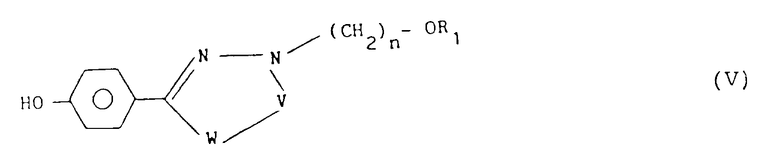 where R₁ and n have the same meaning as in formula (I) and -W-V- represents either -N=N- or 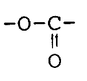 when Ar in formula (IV) is a phenyl ring substituted by R₂, the latter having the same meaning as in formula (I), or -N=N- when Ar in formula (IV) represents pyridyl, in the presence of a base, or
(c) condensation of compounds having the formula: 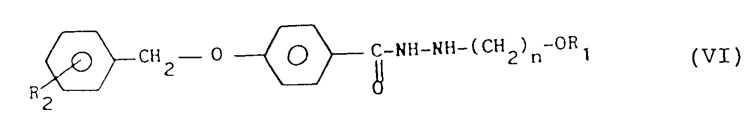 where n, R₁ and R₂ have the same meaning as in formula (I), with phosgene in an aprotic solvent.

8. A process according to claim 7, characterized in that X in formula (II) represents a halogen atom or a mesyloxy or tosyloxy group and the base used in condensation a) is a metal hydride.

9. A process according to claim 7, characterized in that the base used in condensation b) is K₂CO₃ or a metal hydride.

10. Compounds having the formula: 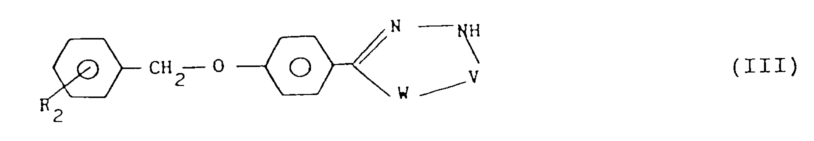 in which -W-V- represents -N=N- or 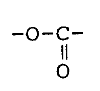 and R₂ represents a hydrogen atom, one or two halogen atoms, a CN, NO₂ or CF₃ group, one, two or three C₁-C₄ alkyl groups or C₁-C₄ alkoxy groups or an amino group substituted by two C₁-C₄ alkyl groups.

11. Compounds having the formula: 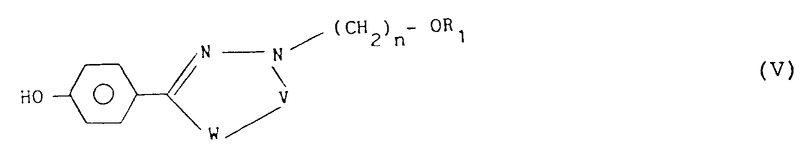 where R₁ = C₁-C₄ alkyl and n = 1-6

12. Compounds having the formula: 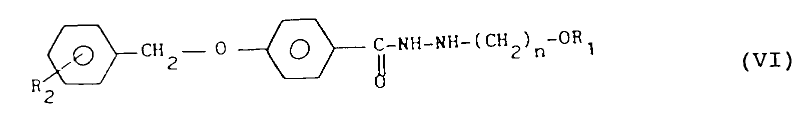 in which n = 2-6, R₁ = C₁-C₄ alkyl and R₂ represents a hydrogen atom, one or two halogen atoms, a CN, NO₂ or CF₃ group, one, two or three C₁-C₄ alkyl groups or C₁-C₄ alkoxy groups or an amino group substituted by two C₁-C₄ alkyl groups.

13. Compounds having the formula: 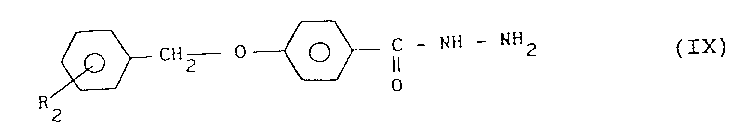 in which R₂ represents a hydrogen atom, one or two halogen atoms, a CN, NO₂ or CF₃ group, one, two or three C₁-C₄ alkyl groups or C₁-C₄ alkoxy groups, or an amino group substituted by two C₁-C₄ alkyl groups, with the exception of 1-[4-benzyloxy benzoyl] hydrazine.

## Patentansprüche

1. Derivate entsprechend der Formel: 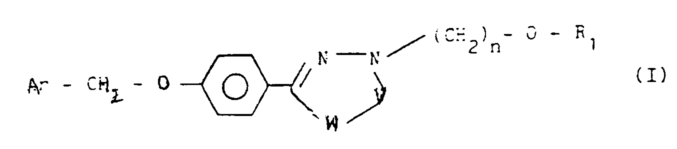 in welcher R₁ = ein Alkyl aus C₁-C₄ und Ar eine Aryl- oder Heteroaryl-Gruppe ist, die unter den folgenden ausgewählt wird:
(i) 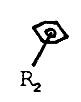 wobei R₂ ein Wasserstoffatom darstellt, ein oder zwei Halogenatome, eine Gruppe CN, NO₂ oder CF₃, ein, zwei oder drei Alkylgruppen aus C₁-C₄ oder Alkoxygruppen aus C₁-C₄, oder eine mit zwei Alkylgruppen aus C₁-C₄ substituierte Aminogruppe, in welchem Fall die Verkettung -W-V- -N=N- oder 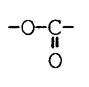 darstellt und n = 2-6 ist;
(ii) Pyridyl, in welchem Fall die Verkettung -W-V- -N=N-darstellt und n= 1-6 ist,
sowie die sauren Additionssalze der salzbildenden Derivate (I).

2. Derivat gemäß Anspruch 1, **dadurch gekennzeichnet**, daß es unter den folgenden ausgewählt wird:
- (Benzyloxy-4 Phenyl)-5 Methoxyethyl-2 2H Tetrazol,
- [(Chloro-4 Benzyloxy)-4 Phenyl]-5 Methoxyethyl-2 2H Tetrazol,
- [(Chloro-3 Benzyloxy)-4 Phenyl]-5 Methoxyethyl-2 2H Tetrazol.

3. [(Chloro-4 Benzyloxy)-4 Phenyl]-5 Methoxyethyl-3 3H Oxadiazol-1,3,4 One-2.

4. Derivat und Salz gemäß Anspruch 1, dadurch gekennzeichnet, daß sie unter den folgenden ausgewählt werden:
- Methoxyethyl-2 [(Pyridyl-4 Methyloxy)-4 Phenyl]-5 2H Tetrazol und sein Chlorhydrat,
- Methoxyethyl-2 [(Pyridyl-3 Methyloxy)-4 Phenyl]-5 2H Tetrazol und sein Chlorhydrat.

5. Pharmazeutische Verbindung, dadurch gekennzeichnet, daß, sie ein Derivat der Formel (I) umfaßt, gemäß einem der Ansprüche 1 bis 4 oder ein pharmazeutisch verwendbares saures Additionssalz eines salzbildenden Derivats der Formel (I) und einen pharmazeutisch verwendbaren Träger.

6. Verwendung von Derivaten und Salzen gemäß einem der Ansprüche 1 bis 4 für die Herstellung eines Medikaments, welches das Monoamin Oxydase vom Typ B hemment.

7. Verfahren zur Herstellung von Derivaten der Formel (I) und Salzen gemäß einem der Ansprüche 1 bis 4, dadurch gekennzeichnet, daß es:
a) die Kondensation der Verbindungen der Formel:
X - (CH₂)ₙ - OR₁ (II)
umfaßt, wobei n = 2-6, R₁ = ein Alkyl aus C₁-C₄, und X also eine leichtabgehende Gruppe darstellt, entsprechend auf den Verbindungen der Formel : 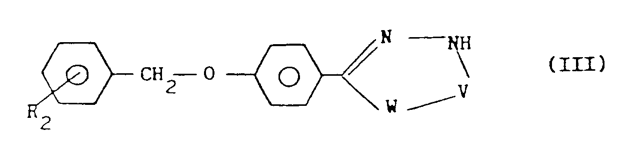 wobei -W-V- -N=N- oder 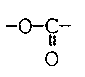 darstellt und R₂ dieselbe Bedeutung hat wie in der Formel (I), in Anwesenheit einer Base,
b) Die Kondensation der Verbindungen der Formel:
Ar - CH₂ - X (IV)
umfaßt, wobei Ar dieselbe Bedeutung hat wie in Formel (I) und X dieselbe Bedeutung hat wie in Formel (II), entsprechend auf den Verbindungen der Formel: 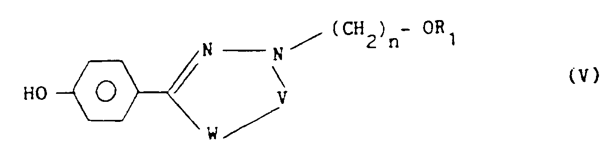 wobei R₁ und n dieselbe Bedeutung haben wie in Formel (I) und -W-V- sowohl -N=N- oder 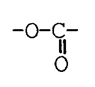 darstellen, wenn Ar in Formel (IV) ein Phenylkern ist, substituiert mit R₂, wobei letztes dieselbe Bedeutung hat wie in Formel (I), als auch -N=N-, wenn Ar in Formel (IV) Pyridyl darstellt, in Anwesenheit einer Base, oder
c) die Kondensation der Verbindungen der Formel: 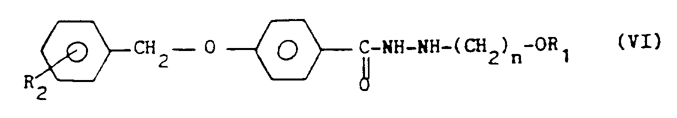 umfaßt, wobei n, R₁ und R₂ dieselbe Bedeutung haben wie in Formel (I), mit Phosgen in einem aprotischen Lösungsmittel.

8. Verfahren gemäß Anspruch 7, dadurch gekennzeichnet, daß, X in Formel (II) ein Halogenatom oder eine Mesyloxy- oder Tosyloxy-Gruppe darstellt, und daß, die in der Kondensation a) eingesetzte Base ein Metallhydrid ist.

9. Verfahren gemäß Anspruch 7, dadurch gekennzeichnet, daß, die in der Kondensation b) eingesetzte Base K₂CO₃ oder ein Metallhydrid ist.

10. Verbindungen der Formel: 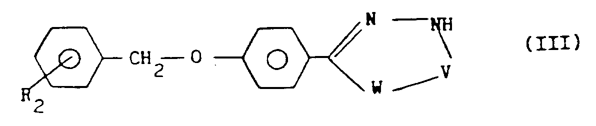 in welcher -W-V- -N=N- oder 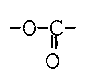 darstellt und R₂ ein Wasserstoffatom, ein oder zwei Halogenatome, eine Gruppe CN, NO₂ oder CF₃, ein, zwei oder drei Alkyl-Gruppen aus C₁-C₄ oder Alkoxy-Gruppen aus C₁-C₄, oder eine mit zwei Alkylgruppen aus C₁-C₄ substituierte Aminogruppe darstellt.

11. Verbindungen der Formel: 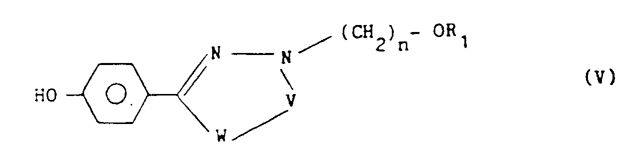 wobei R₁= ein Alkyl aus C₁-C₄ und n = 1-6 ist.

12. Verbindungen der Formel: 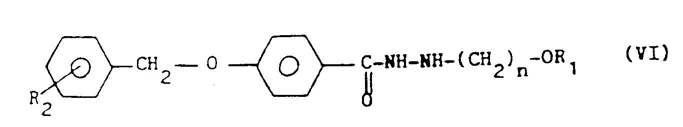 in welcher n = 2-6, R₁ = ein Alkyl aus C₁-C₄ ist und R₂ ein Wasserstoffatom, ein oder zwei Halogenatome, eine Gruppe CN, NO₂ oder CF₃, ein, zwei oder drei Alkylgruppen aus C₁-C₄ oder Alkoxygrupen aus C₁-C₄, oder eine mit zwei Alkylgruppen aus C₁-C₄ substituierte Aminogruppe darstellt.

13. Verbindungen der Formel: 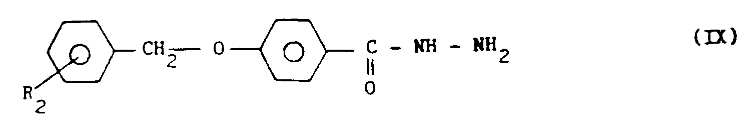 in welcher R₂ ein Wasserstoffatom, ein oder zwei Halogenatome, eine Gruppe CN, NO₂ oder CF₃, ein, zwei oder drei Alkylgruppen aus C₁-C₄ oder Alkoxygrupen aus C₁-C₄, oder eine mit zwei Alkylgruppen aus C₁-C₄ substituierte Aminogruppe, ausgeschlossen [Benzyloxy-4 Benzoyl]-1 Hydrazin, darstellt.
